# EUROPÄISCHE PATENTSCHRIFT

(11) **EP 2 262 798 B1**
(45) Veröffentlichungstag und Bekanntmachung des Hinweises auf die Patenterteilung: **08.06.2016**
(21) Anmeldenummer: 09727209.0
(22) Anmeldetag: 25.03.2009
(51) Int. Cl.: C07D 405/12, C07D 417/12, A01N 43/40, A01N 43/78

(54) **SUBSTITUIERTE ENAMINOTHIOCARBONYLVERBINDUNGEN**
SUBSTITUTED ENAMINOTHIOCARBONYL COMPOUNDS
COMPOSÉS D'ÉNAMINOTHIOCARBONYLE SUBSTITUÉES

(30) Priorität: 31.03.2008 EP 08153687
(43) Veröffentlichungstag der Anmeldung: 22.12.2010
(73) Patentinhaber: Bayer Intellectual Property GmbH, 40789 Monheim am Rhein (DE)
(72) Erfinder: VELTEN, Robert, 40764 Langenfeld (DE); JESCHKE, Peter, 51467 Bergisch Gladbach (DE); FRANKEN, Eva-Maria, 42799 Leichlingen (DE)
(74) Vertreter: BIP Patents
(86) Internationale Anmeldenummer: PCT/EP2009/002164
(87) Internationale Veröffentlichungsnummer: WO 2009/121507

(56) Entgegenhaltungen:
- WO-A-02/085870
- WO-A-2006/037475
- DE-A1-102006 015 467
- DE-A1-102006 015 468

## Beschreibung

Die vorliegende Anmeldung betrifft substituierte Enaminothiocarbonylverbindungen, Verfahren zu Ihrer Herstellung und ihre Verwendung zur Bekämpfung von tierischen Schädlingen, vor allem von Arthropoden und insbesondere von Insekten.

Bestimmte substituierte Enaminothiocarbonylverbindungen sind bereits als insektizid wirksame Verbindungen bekannt geworden (vgl WO 2006/037475 EP 0 539 588 A1) oder wurden als Intermediate zur Herstellung von insektizid wirksamen Verbindungen beschrieben (vgl. WO 2002/085870 A1).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I), 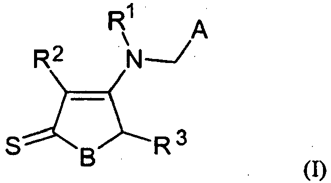 in welcher
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy steht
A für einen Rest 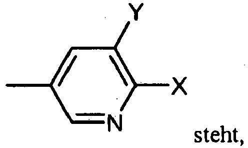 in welchem
   - X: für Halogen, Alkyl oder Halogenalkyl steht
   - Y: für Halogen, Alkyl, Halogenalkyl, Halogenalkoxy, Azido oder Cyan steht,
   - B: für Sauerstoff steht,
   - R¹: Halogenalkyl, oder Halogencycloalkyl steht,
   - R²: für Wasserstoff steht und
   - R³: für Wasserstoff steht,

Weiter wurde gefunden, dass man die neuen substituierten Verbindungen der Formel (I) erhält, wenn man
a) Verbindungen der Formel (II) 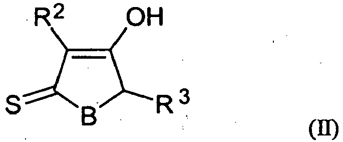 in welcher
   B, R² und R³ die weiter oben genannten Bedeutungen haben
   mit Verbindungen der Formel (III)

      HN(R¹)-CH₂-A (III)

      in welcher
      A und R¹ die weiter oben genannten Bedeutungen haben,
      gegebenenfalls in Gegenwart eines geeigneten Verdünnungsmittels und gegebenenfalls in Gegenwart eines sauren Hilfsmittels umsetzt (Verfahren 1), oder indem man
b) Verbindungen der Formel (Ia) 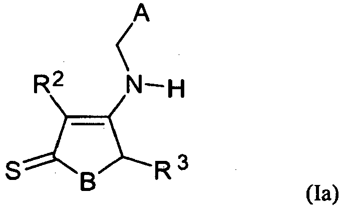 in welcher
   A, B, R² und R³ die weiter oben genannten Bedeutungen haben mit Verbindungen der Formel (IV)

      E-R¹ (IV)

      in welcher
      - R¹: die weiter oben genannten Bedeutungen hat und
      - E: für eine geeignete Abgangsgruppe wie z.B. Halogen (insbesondere Brom, Chlor, Iod) oder O-Sulfonylalkyl und O-Sulfonylaryl (insbesondere O-Mesyl, O-Tosyl) steht,
      gegebenenfalls in Gegenwart eines geeigneten Verdünnungsmittels und gegebenenfalls in Gegenwart eines Säureakzeptors umsetzt (Verfahren 2), oder indem man
c) Verbindungen der Formel (II) 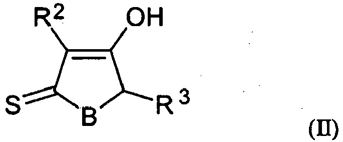 in welcher
   B, R² und R³ die weiter oben genannten Bedeutungen haben,
   in einem ersten Reaktionsschritt, mit Verbindungen der Formel (V)

      H₂N-R¹ (V)

      in welcher
      R¹ die weiter oben genannte Bedeutung hat
   gegebenenfalls in Gegenwart eines geeigneten Verdünnungsmittels und gegebenenfalls in Gegenwart eines sauren Hilfsmittels umsetzt, und anschließend in einem zweiten Reaktionsschritt die gebildeten Verbindungen der Formel (VI) 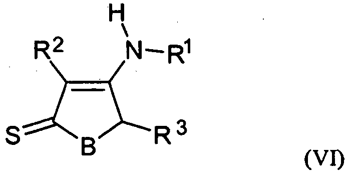 in welcher
      B, R¹, R² und R³ die weiter oben genannten Bedeutungen haben,
      mit Verbindungen der Formel (VII)

         E-CH₂-A (VII)

         in welcher
         E und A die weiter oben genannten Bedeutungen haben
         gegebenenfalls in Gegenwart eines geeigneten Verdünnungsmittels und gegebenenfalls in Gegenwart eines Säureakzeptors umsetzt (Verfahren 3).

Schließlich wurde gefunden, das die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen sind durch die Formel (I) allgemein definiert.

Bevorzugte, besonders bevorzugte und ganz besonders bevorzugte Substituenten bzw. Bereiche der in der oben erwähnten Formel (I) aufgeführten Reste werden im Folgenden erläutert.
- A: steht bevorzugt für 6-Fluor-pyrid-3-yl, 6-Chlor-pyrid-3-yl, 6-Brom-pyrid-3-yl, 6-Methyl pyrid-3-yl, 6-Trifluormethyl-pyrid-3-yl, 6-Trifluormethoxypyrid-3-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-Dichlor-pyrid-3-yl, 5-Brom-6-chlor-pyrid-3-yl, 5-Iod-6-chlorpyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl oder 5-Difluormethyl-6-iod-pyrid-3-yl.
- B: steht für Sauerstoff.
- R¹: steht bevorzugt für gegebenenfalls durch Fluor substituiertes C₁-C₅-Alkyl, C₃-C₅-Cycloalkyl oder C₃-C₅-Cycloalkylalkyl.
- R²: steht für Wasserstoff
- R³: steht bevorzugt für Wasserstoff
- A: steht besonders bevorzugt für den Rest 6-Fluor-pyrid-3-yl, 6-Chlor-pyrid-3-yl, 6-Brompyrid-3-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl oder 5-Difluormethyl-6-chlor-pyrid-3-yl.
- B: steht für Sauerstoff.
- R¹: steht besonders bevorzugt für 2-Fluor-ethyl, 2,2-Difluor-ethyl oder 2-Fluor-cyclopropyl.
- R²: steht für Wasserstoff
- R³: steht für Wasserstoff.
- B: steht für Sauerstoff.
- A: steht ganz besonders bevorzugt für den Rest 6-Chlor-pyrid-3-yl, 6-Brom-pyrid-3-yl, 5-Fluor-6-chlor-pyrid-3-yl oder 5-Fluor-6-brom-pyrid-3-yl.
- R¹: steht ganz besonders bevorzugt für 2-Fluorethyl, oder 2,2-Difluor-ethyl.
- R²: steht für Wasserstoff.
- R³: steht für Wasserstoff.

In einer hervorgehobenen Gruppe von Verbindungen der Formel (I) steht A für 6-Fluor-pyrid-3-yl

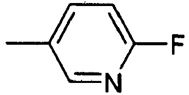

In einer hervorgehobenen Gruppe von Verbindungen der Formel (I) steht A für 6-Chlor-pyrid-3-yl

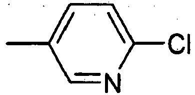

In einer weiteren hervorgehobenen Gruppe von Verbindungen der Formel (I) steht A für 6-Brompyrid-3-yl

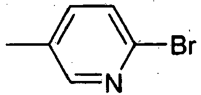

In einer weiteren hervorgehobenen Gruppe von Verbindungen der Formel (I) steht A für 6-Trifluormethyl-pyrid-3-yl 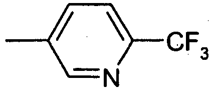
- A: für Pyrid-3-yl, welches in 6-Position durch Fluor, Chlor, Brom, Methyl oder Trifluormethyl substituiert ist steht,
- B: für Sauerstoff,
- R¹: für Halogen-C₁₋₃-alkyl, Halogen-C₂₋₃-alkenyl, Halogencyclopropyl steht (wobei Halogen insbesondere für Fluor oder Chlor steht),
- R²: für Wasserstoff steht,
- R³: für Wasserstoff steht,
- A: bevorzugt für 6-Fluor-pyrid-3-yl, 6-Chlor-pyrid-3-yl, 6-Brom-pyrid-3-yl, 6-Trifluormethyl-pyrid-3-yl, steht,
- B: für Sauerstoff,
- R¹: bevorzugt für Difluormethyl, 2-Fluorethyl, 2,2-Difluorethyl, 2-Chlor-2-fluorethyl, 3-Fluor-*n*-propyl steht,
- R²: bevorzugt für Wasserstoff steht,
- R³: für Wasserstoff steht,
- A: besonders bevorzugt für den Rest 6-Chlor-pyrid-3-yl oder 6-Brom-pyrid-3-yl steht,
- B: für Sauerstoff steht,
- R¹: besonders bevorzugt für 2-Fluorethyl oder 2,2-Difluorethyl steht,
- R²: für Wasserstoff steht,
- R³: für Wasserstoff steht,
- A: ganz besonders bevorzugt für den Rest 6-Chlor-pyrid-3-yl oder 6-Brom-pyrid-3-yl steht,
- B: für Sauerstoff steht,
- R¹: ganz besonders bevorzugt für 2,2-Difluorethyl steht,
- R²: für Wasserstoff steht und
- R³: für Wasserstoff steht.

In einer weiteren hervorgehobenen Gruppe von Verbindungen der Formel (I) stehen R³ für Wasserstoff, B für Sauerstoff und A für 6-Chlor-pyrid-3-yl

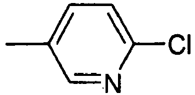

In einer weiteren hervorgehobenen Gruppe von Verbindungen der Formel (I) stehen R³ für Wasserstoff, B für Sauerstoff und A für 6-Brom-pyrid-3-yl

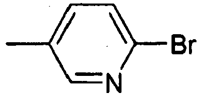

In einer weiteren hervorgehobenen Gruppe von Verbindungen der Formel (I) stehen R³ für Wasserstoff, B für Sauerstoff und A für 6-Fluor-pyrid-3-yl

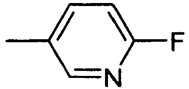

In einer weiteren hervorgehobenen Gruppe von Verbindungen der Formel (I) stehen R³ für Wasserstoff, B für Sauerstoff und A für 6-Trifluormethyl-pyrid-3-yl-

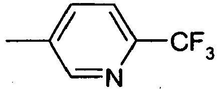

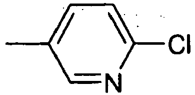

In einer weiteren hervorgehobenen Gruppe von Verbindungen der Formel (I) stehen R² und R³ für Wasserstoff, B für Sauerstoff und A für 6-Brom-pyrid-3-yl

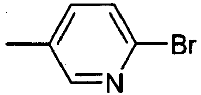

In einer weiteren hervorgehobenen Gruppe von Verbindungen der Formel (I) stehen R² und R³ für Wasserstoff, B für Sauerstoff und A für 6-Fluor-pyrid-3-yl

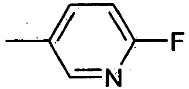

In einer weiteren hervorgehobenen Gruppe von Verbindungen der Formel (I) stehen R² und R³ für Wasserstoff, B für Sauerstoff und A für 6-Trifluormethyl-pyrid-3-yl-

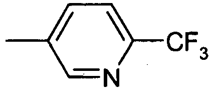

In einer weiteren hervorgehobenen Gruppe von Verbindungen der Formel (I) stehen R¹ für Difluormethyl, R² und R³ für Wasserstoff und B für Sauerstoff.

In einer weiteren hervorgehobenen Gruppe von Verbindungen der Formel (I) stehen R¹ für 2-Fluorethyl, R² und R³ für Wasserstoff und B für Sauerstoff.

In einer weiteren hervorgehobenen Gruppe von Verbindungen der Formel (I) stehen R¹ für 2,2-Difluorethyl, R² und R³ für Wasserstoff und B für Sauerstoff

Die oben aufgeführten allgemeinen oder in Vorzugsbereiche aufgeführten Restedefinitionen bzw. Erläuterungen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Eine bevorzugte Untergruppe der erfindungsgemässen Enaminothiocarbonylverbindungen sind solche der Formel (I-a) 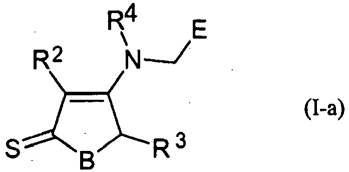 in welcher
E für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy
R⁴ für Halogenalkyl oder Halogencycloalkyl steht, und R², R³ und B die oben angegebenen Bedeutungen haben.

Bevorzugte Substituenten bzw. Bereiche der in der oben und nachstehend erwähnten Formel (I-a) aufgeführten Reste werden im Folgenden erläutert.
- E: steht bevorzugt für 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-y,
- B: steht für Sauerstoff.
- R²: steht für Wasserstoff

- R³: steht für Wasserstoff
- R⁴: steht bevorzugt für durch Fluor substituiertes C₁-C₅-Alkyl, C₃-C₅-Cycloalkyl.
- E: steht besonders bevorzugt für den Rest 6-Fluor-pyrid-3-yl, 6-Chlor-pyrid-3-yl, 6-Brompyrid-3-yl,
- B: steht für Sauerstoff.
- R²: steht für Wasserstoff.
- R³: steht für Wasserstoff.
- R⁴: steht besonders bevorzugt für 2-Fluor-ethyl, 2,2-Difluor-ethyl, 2-Fluor-cyclopropyl.
- E: steht ganz besonders bevorzugt für den Rest 6-Chlor-pyrid-3-yl, 6-Brom-pyrid-3-yl, oder
- B: steht für Sauerstoff.
- R²: steht für Wasserstoff.
- R³: steht für Wasserstoff.
- R⁴: steht für 2,2-Difluor-ethyl.

In einer hervorgehobenen Gruppe von Verbindungen der Formel (I-a) steht E für 6-Chlor-pyrid-3-yl

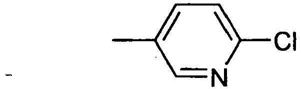

In einer weiteren hervorgehobenen Gruppe von Verbindungen der Formel (I-a) steht E für 6-Brom-pyrid-3-yl

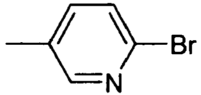

Im Folgenden ist eine weitere Gruppe bevorzugter Verbindungen der Formel (I-a) definiert, in welcher
- E: für Pyrid-3-yl, welches in 6-Position durch Fluor, Chlor, Brom, Methyl oder Trifluormethyl substituiert ist steht,
- B: für Sauerstoff steht,
- R²: für Wasserstoff steht,
- R³: für Wasserstoff steht,
- R⁴: für Halogen-C₁₋₃-alkyl, Halogencyclopropyl steht (wobei Halogen insbesondere für Fluor oder Chlor steht),
- E: bevorzugt für 6-Fluor-pyrid-3-yl, 6-Chlor-pyrid-3-yl, 6-Brom-pyrid-3-yl, 6-Trifluormethyl-pyrid-3-yl, steht,
- B: für Sauerstoff steht,
- R²: für Wasserstoff,

- R³: für Wasserstoff steht,
- R⁴: bevorzugt für Difluormethyl, 2-Fluorethyl, 2,2-Difluorethyl, 2-Chlor-2-fluorethyl, 3-Fluor-*n*-propyl, steht,
- E: besonders bevorzugt für den Rest 6-Chlor-pyrid-3-yl oder 6-Brom-pyrid-3-yl steht,
- B: für Sauerstoff steht,
- R²: für Wasserstoff steht,
- R³: für Wasserstoff steht,
- R⁴: besonders bevorzugt für 2-Fluorethyl oder 2,2-Difluorethyl steht,
- E: ganz besonders bevorzugt für den Rest 6-Chlor-pyrid-3-yl oder 6-Brom-pyrid-3-yl steht,
- B: für Sauerstoff,
- R²: für Wasserstoff steht,
- R³: für Wasserstoff steht und
- R⁴: ganz besonders bevorzugt für 2,2-Difluorethyl steht.

In einer hervorgehobenen Gruppe von Verbindungen der Formel (I-a) stehen R³ für Wasserstoff, B für Sauerstoff und E für 6-Chlor-pyrid-3-yl

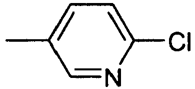

In einer weiteren hervorgehobenen Gruppe von Verbindungen der Formel (I-a) stehen R³ für Wasserstoff, B für Sauerstoff und E für 6-Brom-pyrid-3-yl

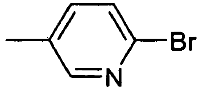

In einer weiteren hervorgehobenen Gruppe von Verbindungen der Formel (I-a) stehen R³ für Wasserstoff, B für Sauerstoff und E für 6-Fluor-pyrid-3-yl

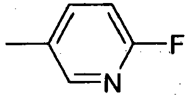

In einer weiteren hervorgehobenen Gruppe von Verbindungen der Formel (I-a) stehen R³ für Wasserstoff, B für Sauerstoff und E für 6-Trifluormethyl-pyrid-3-yl-

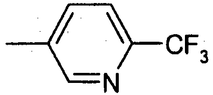

In einer weiteren hervorgehobenen Gruppe von Verbindungen der Formel (I-a) stehen R² und R³ für Wasserstoff, B für Sauerstoff und E für 6-Chlor-pyrid-3-yl

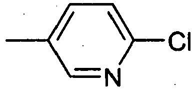

In einer weiteren hervorgehobenen Gruppe von Verbindungen der Formel (I-a) stehen R² und R³ für Wasserstoff, B für Sauerstoff und E für 6-Brom-pyrid-3-yl

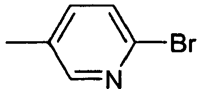

In einer weiteren hervorgehobenen Gruppe von Verbindungen der Formel (I-a) stehen R² und R³ für Wasserstoff, B für Sauerstoff und E für 6-Fluor-pyrid-3-yl

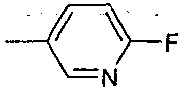

In einer weiteren hervorgehobenen Gruppe von Verbindungen der Formel (I-a) stehen R² und R³ für Wasserstoff, B für Sauerstoff und E für 6-Trifluormethyl-pyrid-3-yl-

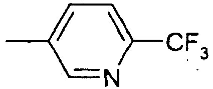

In einer weiteren hervorgehobenen Gruppe von Verbindungen der Formel (I-a) stehen R⁴ für Difluormethyl, R² und R³ für Wasserstoff und B für Sauerstoff.

In einer weiteren hervorgehobenen Gruppe von Verbindungen der Formel (I-a) stehen R⁴ für 2-Fluorethyl, R² und R³ für Wasserstoff und B für Sauerstoff.

In einer weiteren hervorgehobenen Gruppe von Verbindungen der Formel (I-a) stehen R⁴ für 2,2-Difluorethyl, R² und R³ für Wasserstoff und B für Sauerstoff.

Eine weitere bevorzugte Untergruppe der erfindungsgemässen Enaminothiocarbonylverbindungen sind solche der Formel (I-c) 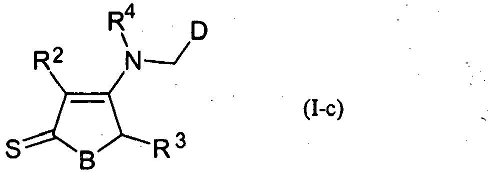 in welcher
D für einen Rest 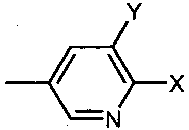 steht in welchem
X und Y die oben angegebenen Bedeutungen haben,
R⁴ für Halogenalkyl, Halogencycloalkyl steht,
und R², R³ und B die oben angegebenen Bedeutungen haben.

Bevorzugte Substituenten bzw. Bereiche der in der oben und nachstehend erwähnten Formel (I-c) aufgeführten Reste werden im Folgenden erläutert.
- D: bevorzugt für einen der Reste 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-Dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl, 5-Difluormethyl-6-iod-pyrid-3-yl.
- B: steht für Sauerstoff.
- R².: steht für Wasserstoff

- R³: steht für Wasserstoff.
- R⁴: steht bevorzugt für durch Fluor substituiertes C₁-C₅-Alkyl, C₃-C₅-Cycloalkyl.
- D: steht besonders bevorzugt für, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl oder 5-Difluormethyl-6-chlor-pyrid-3-yl.
- B: steht für Sauerstoff.
- R²: steht für Wasserstoff.
- R³: steht für Wasserstoff.
- R⁴: steht besonders bevorzugt für 2-Fluor-ethyl, 2,2-Difluor-ethyl, 2-Fluor-cyclopropyl.
- D: steht ganz besonders bevorzugt für 5-Fluor-6-chlor-pyrid-3-yl oder 5-Fluor-6-brom-pyrid-3-yl.
- B: steht für Sauerstoff.
- R²: steht für Wasserstoff.
- R³: steht für Wasserstoff.
- R⁴: steht ganz besonders bevorzugt für 2,2-Difluor-ethyl.

In einer weiteren hervorgehobenen Gruppe von Verbindungen der Formel (I-c) stehen R³ für Wasserstoff, B für Sauerstoff und D für 5-Fluor-6-chlor-pyrid-3-yl,

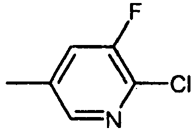

In einer weiteren hervorgehobenen Gruppe von Verbindungen der Formel (I-c) stehen R³ für Wasserstoff, B für Sauerstoff und D für 5,6-Dichlor-pyrid-3-yl

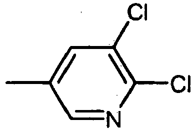

In einer weiteren hervorgehobenen Gruppe von Verbindungen der Formel (I-c) stehen R³ für Wasserstoff, B für Sauerstoff und D für 5-Brom-6-chlor-pyrid-3-yl

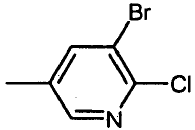

In einer weiteren hervorgehobenen Gruppe von Verbindungen der Formel (I-c) stehen R³ für Wasserstoff, B für Sauerstoff und D für 5-Methyl-6-chlor-pyrid-3-yl

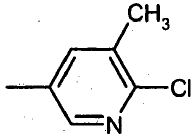

In einer weiteren hervorgehobenen Gruppe von Verbindungen der Formel (I-c) stehen R³ für Wasserstoff, B für Sauerstoff und D für 5-Fluor-6-brom-pyrid-3-yl

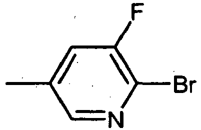

In einer weiteren hervorgehobenen Gruppe von Verbindungen der Formel (I-c) stehen R³ für Wasserstoff, B für Sauerstoff und D für 5-Chlor-6-brom-pyrid-3-yl

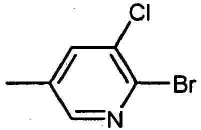

In einer weiteren hervorgehobenen Gruppe von Verbindungen der Formel (I-c) stehen R³ für Wasserstoff, B für Sauerstoff und D für 5-Chlor-6-iod-pyrid-3-yl

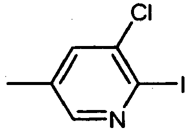

In einer weiteren hervorgehobenen Gruppe von Verbindungen der Formel (I-c) stehen R² und R³ für Wasserstoff, B für Sauerstoff und D für 5-Fluor-6-chlor-pyrid-3-yl,

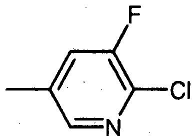

In einer weiteren hervorgehobenen Gruppe von Verbindungen der Formel (I-c) stehen R² und R³ für Wasserstoff, B für Sauerstoff und D für 5,6-Dichlor-pyrid-3-yl

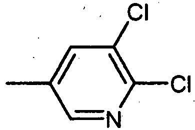

In einer weiteren hervorgehobenen Gruppe von Verbindungen der Formel (I-c) stehen R² und R³ für Wasserstoff, B für Sauerstoff und D für 5-Brom-6-chlor-pyrid-3-yl

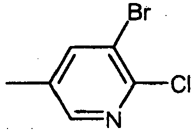

In einer weiteren hervorgehobenen Gruppe von Verbindungen der Formel (I-c) stehen R² und R³ für Wasserstoff, B für Sauerstoff und D für 5-Methyl-6-chlor-pyrid-3-yl

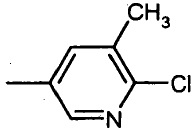

In einer weiteren hervorgehobenen Gruppe von Verbindungen der Formel (I-c) stehen R² und R³ für Wasserstoff, B für Sauerstoff und D für 5-Fluor-6-brom-pyrid-3-yl

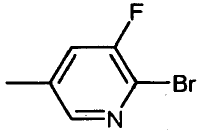

In einer weiteren hervorgehobenen Gruppe von Verbindungen der Formel (I-c) stehen R² und R³ für Wasserstoff, B für Sauerstoff und D für 5-Chlor-6-brom-pyrid-3-yl

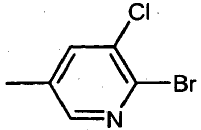

In einer weiteren hervorgehobenen Gruppe von Verbindungen der Formel (I-c) R² und R³ für Wasserstoff, B für Sauerstoff und D für 5-Chlor-6-iod-pyrid-3-yl

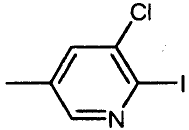

In einer weiteren hervorgehobenen Gruppe von Verbindungen der Formel ,(I-c) stehen R⁴ für Difluormethyl, R² und R³ für Wasserstoff und B für Sauerstoff.

In einer weiteren hervorgehobenen Gruppe von Verbindungen der Formel (I-c) stehen R⁴ für 2-Fluorethyl, R² und R³ für Wasserstoff und B für Sauerstoff.

In einer weiteren hervorgehobenen Gruppe von Verbindungen der Formel (I-c) stehen R⁴ für 2,2-Difluorethyl, R² und R³ für Wasserstoff und B für Sauerstoff

Im Einzelnen seien die folgenden Verbindungen der allgemeinen Formel (I) genannt:
- Verbindung (I-1), 4-{[(6-Brompyrid-3-yl)methyl](2-fluorethyl)amino}furan-2(5H)-thion, besitzt die Formel: 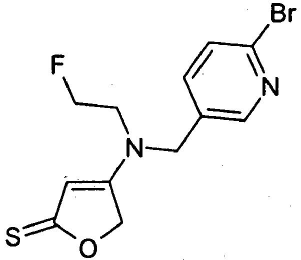
- Verbindung (I-2), 4-{[(6-Br6mpyrid-3-yl)methyl](2,2-difluorethyl)amino}furan-2(5H)-thion, besitzt die Formel 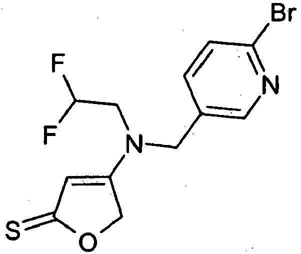
- Verbindung (I-3), 4-{[(6-Fluorpyrid-3-yl)methyl](2,2-difluorethyl)amino}furan-2(5H)-thion, besitzt die Formel 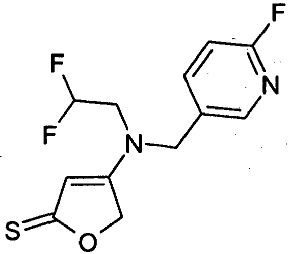
- Verbindung (I-4), 4-{[(2-Chlor-1,3-thiazol-5-yl)methyl](2-fluorethyl)amino}furan-2(5H)-thion, besitzt die Formel: 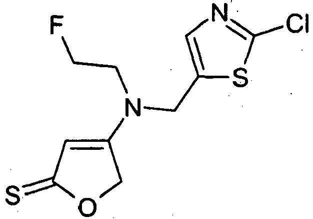
- Verbindung (I-5), 3-Chlor-4-{[(6-chlorpyrid-3-yl)methyl](2-fluorethyl)amino}furan-2(5H)-thion, besitzt die Formel 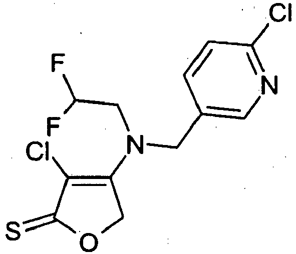
- Verbindung (I-6), 4-{[(6-Chlorpyrid-3-yl)methyl](2-fluorethyl)amino}furan-2(5H)-thion, besitzt die Formel 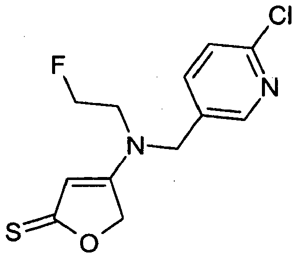
- Verbindung (I-7), 4-{[(6-Chlorpyrid-3-yl)methyl](2,2-difluorethyl)amino}furan-2(5H)-thion, besitzt die Formel 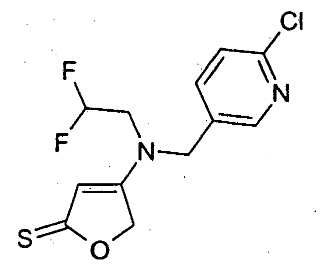
- Vergleichsverbindung (1-8) 4-{[(6-Chlor-5-fluorpyrid-3-yl)methyl](methyl)amino} furan-2(5H)-thion, besitzt die Formel 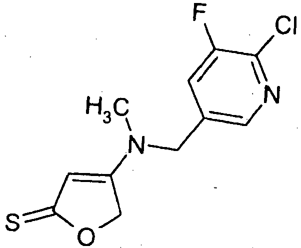
- Vergleichsverbindung (I-9), 4-{[(5,6-Dichlorpyrid-3-yl)methyl](cyclopropyl)amino}furan-2(5H)-thion, besitzt die Formel 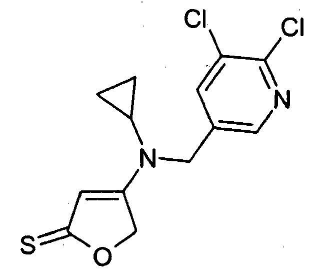
- Vergleichsverbindung (I-10), 4-{[(5,6-Dichlorpyrid-3-yl)methyl](methyl)amino}furan-2(5H)-thion, besitzt die Formel 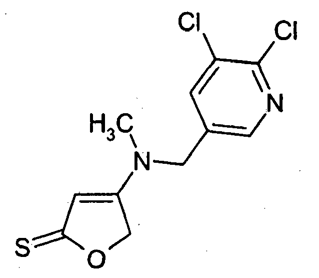
- Vergleichsverbindung (I-11), 4-{[(6-Brom-5-fluorpyrid-3-yl)methyl](methyl)amino}furan-2(5H)-thion, besitzt die Formel 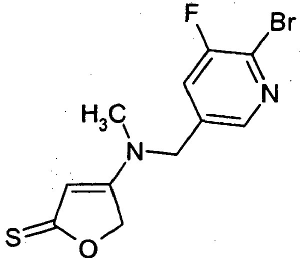
- Vergleichsverbindung (I-12), 4-{[(6-Brom-5-fluorpyrid-3-yl)methyl](cyclopropyl)amino}furan-2(5H)-thion, besitzt die Formel 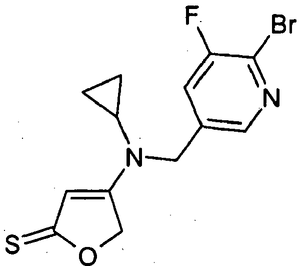
- Vergleichsverbindung (I-13), 4-{[(6-Chlor-5-fluorpyrid-3-yl)methyl](cyclopropyl)amino}furan-2(5H)-thion, besitzt die Formel 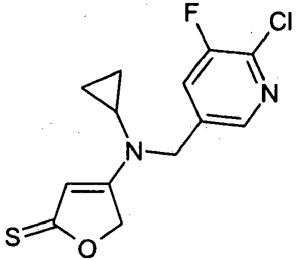
- Verbindung (I-14), 4-{[(5,6-Dichlorpyrid-3-yl)methyl](2-fluorethyl)amino}furan-2(5H)-thion, besitzt die Formel 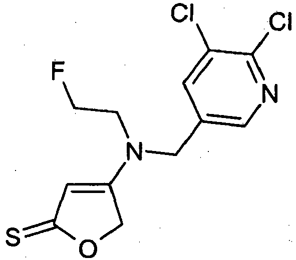
- Verbindung (I-15), 4-{[(5,6-Dichlorpyrid-3-yl)methyl](2-fluorethyl)amino}furan-2(5H)-thion, besitzt die Formel 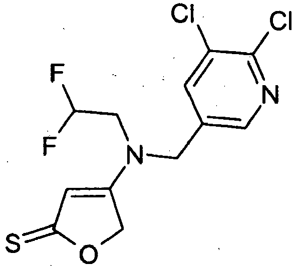
- Verbindung (1-16), 4-{[(6-Brom-5-fluorpyrid-3-yl)methyl](2,2-difluorethyl)amino}furan-2(5H)-thion, besitzt die Formel 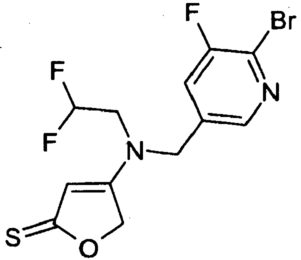
- Verbindung (1-17), 4-{[(6-Chlor-5-fluorpyrid-3-yl)rnethyl](2,2-difluorethyl)amino}furan-2(5H)-thion, besitzt die Formel 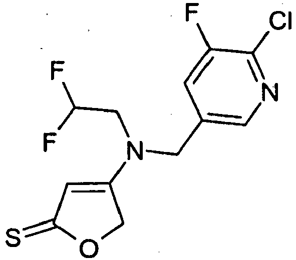
- Vergleichsverbindung (I-18), 4-{[(2-Chlor-2,3-dihydro-1,3-thiazol-5-yl)methyl](methyl) amino}furan-2(5H)-thion, besitzt die Formel 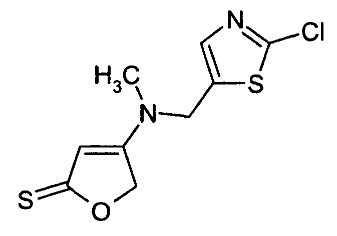
- Vergleichsverbindung (I-19), 4-[Methyl(pyrid-3-ylmethyl)amino]furan-2(5H)-thion, besitzt die Formel 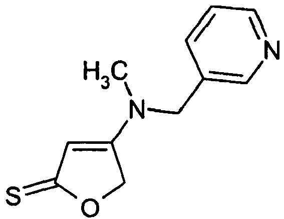
- Vergleichsverbindung (I-20), 4-{Cyclopropyl[(6-fluorpyrid-3-yl)methyl]amino}furan-2(5H)-thion, besitzt die Formel 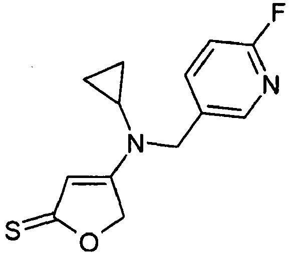
- Vergleichsverbindung (I-21), 4-(Methyl{[6-(trifluormethyl)pyrid-3-yl]methyl}amino)furan-2(5H)-thion, besitzt die Formel 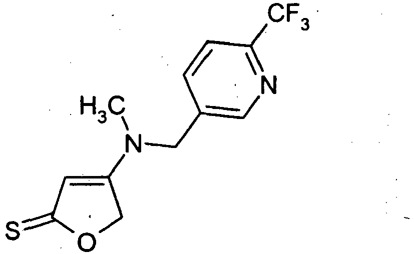
- Vergleichsverbindung (I-22), 4-(Cyclopropyl{[6-(trifluormethyl)pyrid-3-yl]methyl}amino)furan-2(5H)-thion, besitzt die Formel 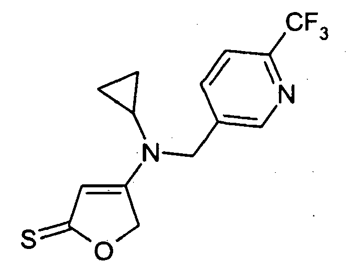
- Vergleichsverbindung (I-23), 4-{[(6-Chloropyrid-3-yl)methyl](methyl)amino}-5-methylfuran-2(5H)-thion, besitzt die Formel 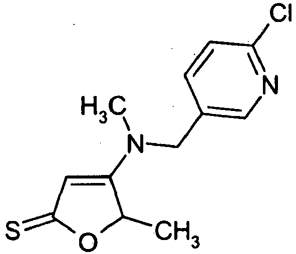
- Vergleichsverbindung (I-24), 4-{[(6-Brompyrid-3-yl)methyl](methyl)amino}furan-2(5H)-thion, besitzt die Formel 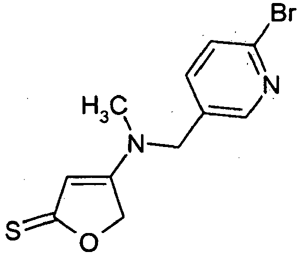
- Vergleichsverbindung (I-25), 4-{[(6-Chlorpyrid-3-yl)methyl](methyl)amino}-3-fluorofuran-2(5H)-thion, besitzt die Formel 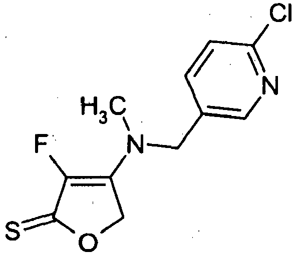
- Vergleichsverbindung (I-26), 4-{[(6-Chloropyrid-3-yl)methyl](methoxy)amino}furan-2(5H)-thion, besitzt die Formel 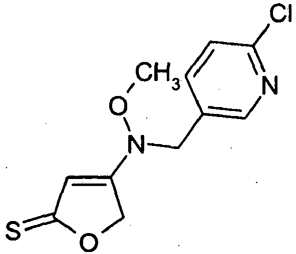
- Vergleichsverbindung (I-27), 4-{[(6-Chloropyrid-3-yl)methyl](ethyl)amino}furan-2(5H)-thion, besitzt die Formel 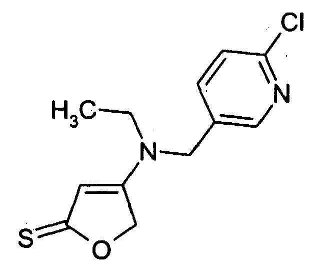
- Vergleichsverbindung (I-28), 4-{[(6-Chlorpyrid-3-yl)methyl](cyclopropyl)amino}furan-2(5H)-thion, besitzt die Formel 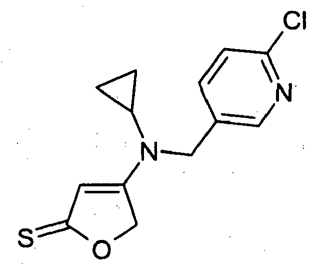
- Vergleichsverbindung (I-29), 4-{Allyl[(6-chlorpyrid-3-yl)methyl]amino}furan-2(5H)-thion besitzt die Formel 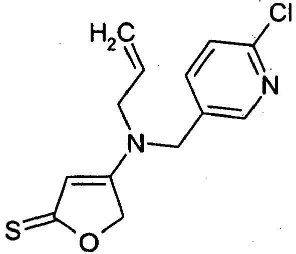
- Vergleichsverbindung (I-30), 4-{[(6-Chlorpyrid-3-yl)methyl](prop-2-in-1-yl)amino}furan-2(5H)-thion, besitzt die Formel

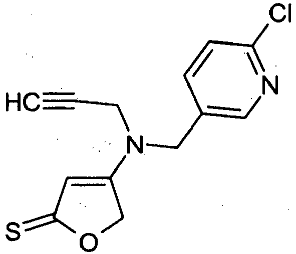

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt oder ganz besonders bevorzugt sind Verbindungen, welche jeweils die unter bevorzugt, besonders bevorzugt oder ganz besonders bevorzugt genannten Substituenten tragen.

Gesättigte Kohlenwasserstoffreste wie Alkyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Setzt man bei dem erfindungsgemäßen Verfahren 1 zur Herstellung der neuen Verbindungen der Formel (I) als Verbindung der Formel (II) beispielsweise die 4-Hydroxyfuran-2(5*H*)-thion ein und als Verbindung der Formel (III) das *N*-[6-Chlorpyridin-3-yl)methyl]-2,2-difluorethan-1-amin ein, so lässt sich das Herstellungsverfahren 1 durch folgendes Reaktionsschema I wiedergeben:

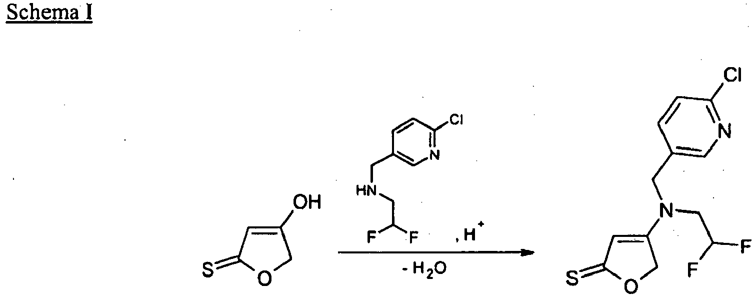

Die zur Durchführung des erfindungsgemäßen Verfahrens 1 als Ausgangsstoffe benötigten Verbindungen sind durch die Formel (II) allgemein definiert.

In dieser Formel (II) stehen B, R² und R³ vorzugsweise für diejenigen Reste, die bereits im Zusammenhang mit der Beschreibung der erfindungsgemäßen Stoffe der Formel (I) als bevorzugte Substituenten genannt werden.

Die Verbindungen der Formel (II) können nach literaturbekannten Methoden erhalten werden (vgl. Schema II, z. B. für Verbindungen der allgemeinen Formel II, in welcher B für Sauerstoff und R¹, R² jeweils für Wasserstoff stehen: 4-Hydroxyfuran-2(5*H*)-thion (R. Labruère et al., Synthesis 24, 4163-4166, 2006) und B für Schwefel und R¹, R² jeweils für Wasserstoff stehen: 4-Hydroxythiophen-2(5*H*)-thion (R. Labruère et al., Synthesis 24, 4163-4166, 2006).

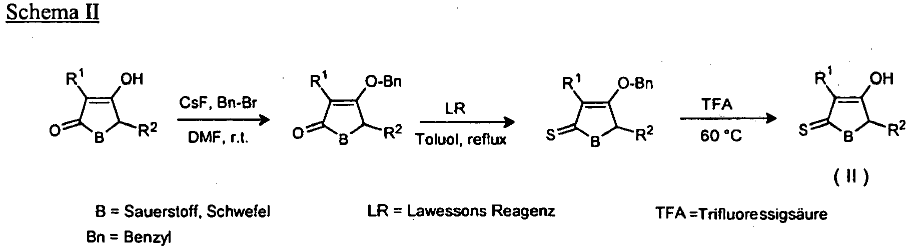

Die weiteren für die Durchführung des erfindungsgemäßen Verfahrens 1 als Ausgangsstoffe zu verwendenden Verbindungen sind allgemein durch die Formel (III) definiert.

In der Formel (III) haben A und R¹ die Bedeutungen, die bereits in Zusammenhang mit der Beschreibung der erfindungsgemäßen Stoffe der Formel (I) genannt wurden.

Die Verbindungen der Formel (III) können z. T. kommerziell oder nach literaturbekannten Methoden erhalten werden (vgl. z. B. S. Patai "The Chemistry of Amino Group", Interscience Publishers, New York, 1968; Verbindungen der allgemeinen Formel (III), in welcher R¹ für Wasserstoff steht: primäre Amine, R¹ für Halogenalkyl, Halogenalkenyl oder Halogencycloalkyl steht: sekundäre Amine).

Die Verbindungen der Formel (III) lassen sich auch aus Verbindungen der Formel (VII) herstellen (vgl. Schema III weiter unten).

Die Verbindungen der Formel (VII) sind z. T. kommerziell erhältlich, teilweise bekannt und können nach bekannten Methoden erhalten werden (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 6-Methyl-3-chlormethyl-pyridin: EP 302389 A2, E. v der Eycken et al., J. Chem. Soc., Perkin Trans 2 5, 928-937 (2002); 6-Trifluormethyl-3-chlormethyl-pyridin: WO 2004/082616 A2; 2-Chlor-5chlormethyl-pyrazin: JP 05239034 A2).

Allgemeine Wege zur Herstellung von Verbindungen der Formel (VII) sind im Schema III wiedergegeben.

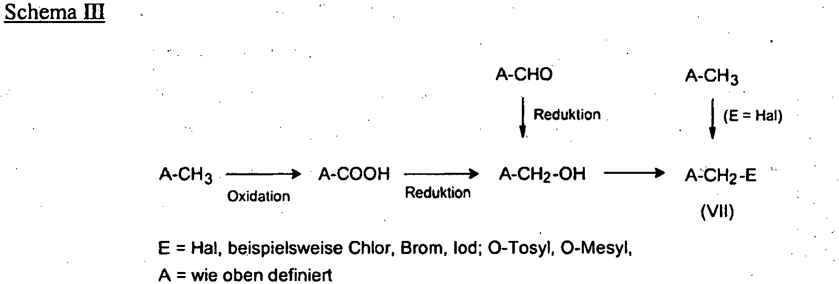

Beispielsweise können die heterocyclischen Carbonsäuren (A-COOH) nach literaturbekannten Methoden in die entsprechenden heterocyclischen Hydroxymethylverbindungen (A-CH₂-OH) überführt werden, die anschließend nach literaturbekannten Methoden zu aktivierten heterocyclischen Hydroxymethylverbindungen (A-CH₂-E, E = OTosyl, OMesyl) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Zur Darstellung von Verbindungen der Formel (III) ist es vorteilhaft, dass man beispielsweise Verbindungen der Formel (VII), in der A und E die weiter oben genannte Bedeutung haben, mit Verbindungen der Formel (V), in der R¹ die weiter oben genannte Bedeutung hat, gegebenenfalls in Gegenwart von Verdünnungsmitteln und gegebenenfalls in Gegenwart von den im Herstellungsverfahren 2 genannten basischen Reaktionshilfsmitteln umsetzt (vgl. *N*-Alkylierung, Schema IV).

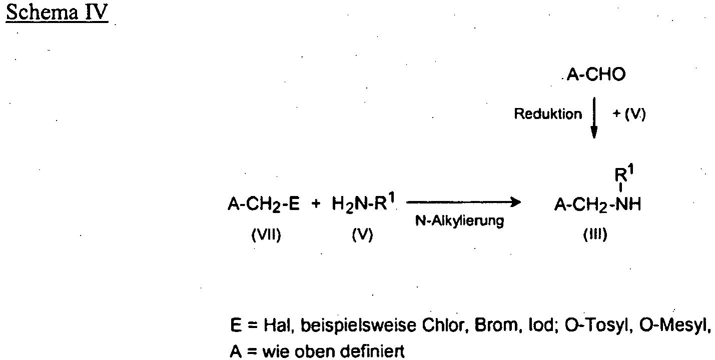

Die Verbindungen der Formel (V) können z. T. kommerziell (vgl. z. B. 2-Fluorethylamin oder 2,2-Difluorethylamin) oder nach literaturbekannten Methoden erhalten werden (vgl. z. B. 3-Fluor-*n-*propylamin: US 6252087 B1; 3,3-Difluor-prop-2-enylamin Hydrochlorid: WO 2001/007414 A1; 3,3-Dichlor-prop-2-enylamin: DE 2747814).

Alternativ und in bestimmten Fällen ist aber auch eine Herstellung von Verbindungen der Formel (III) aus den entsprechenden Aldehyden (A-CHO) und Verbindungen der Formel (V) mittels reduktiver Aminierung möglich (vgl. Houben-Weyl, Methoden der Organischen Chemie, Bd. XI/1, Georg Thieme Verlag Stuttgart, S. 602). Die Aldehyde (A-CHO) sind z. T. kommerziell erhältlich (vgl. z. B. 6-Chlor-nicotinaldehyd, 6-Fluor-nicotinaldehyd, 6-Brom-nicotinaldehyd, 2-Chlor-1,3-thiazol-5-carbaldehyd) oder können nach literaturbekannten Methoden erhalten werden (vgl. z. B. 6-Methyl-nicotinaldehyd: EP 104876 A2; 2-Chlor-pyrazin-5-carboxaldehyd: DE 3314196 A1).

Die Darstellung der Verbindungen der allgemeinen Formel (III) ist beispielsweise auch in WO 2007/115644, WO 2007/115646 oder WO 2008/009360 A2 beschrieben und kann in analoger Weise erfolgen.

Im Allgemeinen ist es vorteilhaft, das erfindungsgemäße Herstellungsverfahren 1 in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 1 können alle unter den Reaktionsbedingungen inerten organischen Lösungsmittel in Frage kommen. Diese Verdünnungsmittel sind in der Beschreibung des erfindungsgemäßen Verfahrens 2 genannt.

Selbstverständlich kann man in das erfindungsgemäße Verfahren 1 auch in Gemischen der genannten Lösungs- und Verdünnungsmittel durchführen.

Die Herstellung von Verbindungen der Formel (I) nach dem Herstellungsverfahren 1 wird bevorzugt durchgeführt, indem man Verbindungen der Formel (II) in Gegenwart von Verbindungen der Formel (III), gegebenenfalls in Gegenwart eines sauren Hilfsmittels und gegebenenfalls in einem der angegebenen Verdünnungsmittel umsetzt.

Als saure Hilfsmittel sind beispielsweise anorganische Säuren wie Salzsäure, Bromwassetstoffsäure, Salpetersäure, Schwefelsäure, Phosphorsäure, oder organische Säuren wie Ameisensäure, Essigsäure, Propionsäure, Malonsäure, Oxalsäure, Fumarsäure, Adipinsäure, Stearinsäure, Weinsäure, Milchsäure, Ölsäure, Methansulfonsäure, Benzolsulfonsäure oder Toluolsulfonsäure bekannt.

Die Herstellung von Verbindungen der Formel (I) nach dem Herstellungsverfahren 1 wird besonders bevorzugt durchgeführt, indem man Verbindungen der Formel (II) in Gegenwart von Verbindungen der Formel (III) in einem sauren Hilfsmittels, das gleichzeitig ein Verdünnungsmittel ist, umsetzt.

Als geeignetes saures Hilfsmittels, das gleichzeitig Verdünnungsmittel ist, kann beispielsweise Essigsäure verwendet werden.

Die Reaktionsdauer beträgt im Allgemeinen 10 Minuten bis 20 Tage. Die Umsetzung erfolgt bei Temperaturen zwischen -10°C und +150°C, bevorzugt zwischen +10°C und 100°C, besonders bevorzugt bei Raumtemperatur.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Verfahrens 1 setzt man pro Mol Verbindung der allgemeinen Formel (II) im Allgemeinen 0,5 bis 5,0 Mol, vorzugsweise 0,7 bis 3,5 Mol, besonders bevorzugt 1,0 bis 3,0 Mol an Aminoverbindung der allgemeinen Formel (III) ein.

Alternativ kann man aber auch durch Auswahl eines geeigneten Lösungs- und Verdünnungsmittels (genannt unter Verfahren 2) bevorzugt unter Reaktionsbedingungen arbeiten, die das Abscheiden oder Entfernen von Wasser ermöglichen, beispielsweise unter Zuhilfenahme eines Wasserabscheiders.

Bevorzugte Verdünnungsmittel zur Durchführung des erfindungsgemäßen Verfahrens 1 sind aromatische Kohlenwasserstoffe wie Benzol, Toluol, Chlorbenzol, Brombenzol, Nitrobenzol oder Xylol, insbesondere Benzol und Toluol.

Weiterhin kann man zur Durchführung des erfindungsgemäßen Verfahrens 1 in einem organischen Lösungsmittel im Allgemeinen katalytische Mengen eines weiter oben genannten sauren Hilfsmittels hinzusetzen.

Als saure Hilfsmittel kommen beispielsweisep-Toluolsulfonsäure oder Essigsäure in Frage.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Verfahren 2 zur Herstellung der neuen Verbindungen der Formel (I) als Verbindung der Formel (Ia) beispielsweise 4-[[(6-Chlor-pyridin-3-yl)methyl]-amino]furan-2(5H)-thion und als Verbindung der Formel (IV) das 3-Brom-1,1-dichlor-prop-1-en ein, so lässt sich das Herstellungsverfahren 2 durch folgendes Reaktionsschema V wiedergeben: 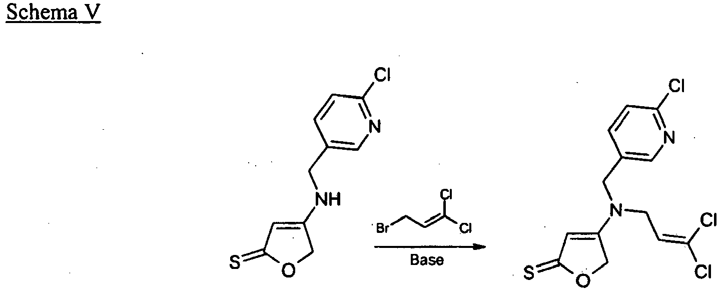 Die zur Durchführung des erfindungsgemäßen Verfahrens 2 als Ausgangsstoffe benötigten Verbindungen sind durch die Formel (la) allgemein definiert.

In dieser Formel (Ia) stehen A, B, R² und R³ vorzugsweise für diejenigen Reste, die bereits im Zusammenhang mit der Beschreibung der erfindungsgemäßen Stoffe der Formel (I) als bevorzugte Substituenten genannt werden.

Die Verbindungen der Formel (la) können nach dem weiter oben beschriebenen Herstellverfahren 1 erhalten werden, beispielsweise durch Reaktion von Verbindungen der Formel (II) mit Verbindungen der Formel (111), in welcher R¹ für Wasserstoff steht.

Das insbesondere für die Durchführung des erfindungsgemäßen Verfahrens 2 als Ausgangsstoff verwendete 4-[[(6-Chlor-pyridin-3-yl)methyl]amino]furan-2(5H)-thion ist aus EP 0 539 588 A1 bekannt.

Die insbesondere für die Durchführung des erfindungsgemäßen Verfahrens 2 als Ausgangsstoffe zu verwendenden Verbindungen sind allgemein durch die Formel (IV) definiert.

In der Formel (IV) haben E und R¹ die Bedeutung die bereits im Zusammenhang mit der Beschreibung der erfindungsgemäßen Stoffe der allgemeinen Formel (I) genannt wurde.

Die Verbindungen der Formel (IV) können z. T. kommerziell erhalten werden (vgl. z. B. Chlordifluormethan, 1-Brom-2-fluorethan, 2-Brom-1,1-difluorethan, 2-Brom-1-chlor-1-fluorethan, 1-Brom-3-fluorpropan, 3-Brom-1,1-difluor-prop-1-en, oder nach literaturbekannten Methoden erhalten werden (vgl. z. B. 3-Brom-1,1-dichlor-prop-1-en: WO 8800183 A1 (1988); Verbindungen der allgemeinen Formel IV in welcher E für Halogen wie Chlor, Brom und Iod steht: Houben-Weyl, Methoden der Organischen Chemie, Bd, V/3, Georg Thieme Verlag Stuttgart, S. 503 und Bd. V/4 S. 13, 517; E' für Mesylat steht: Crossland, R. K., Servis, K. L. J. Org. Chem. (1970), 35, 3195; E für Tosylat steht: Roos, A. T. et al., Org. Synth., Coll. Vol. I, (1941), 145; Marvel, C. S., Sekera, V.C.Org.Synth.,Coll.Vol.III,(1955),366.

Im Allgemeinen ist es vorteilhaft, das erfindungsgemäße Herstellungsverfahren 2 in Gegenwart von Verdünnungsmitteln und in Gegenwart von basischen Reaktionshilfsmitteln durchzuführen.

Verdünnungsmittel werden vorteilhaft in einer solchen Menge eingesetzt, dass das Reaktionsgemisch während des ganzen Verfahrens gut rührbar bleibt. Als Verdünnungsmittel zur Durchfüh-rung des erfindungsgemäßen Verfahrens 2 kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-bütylether, *n*-Butylether, Anisol, Phenetol, Cyclohexylmethylether, Dimethylether, Diethylether, Diproplether, Diisopropylether, Di-*n*-butylether, Diisobutylether, Diisoamylether, Ethylenglycoldimethylether, Tetrahydrofuran, Dioxan, Dichlordiethylether und Polyether des Ethylenoxids und/oder Propyle-noxids; Amine wie Trimethyl-, Triethyl-, Tripropyl-, Tributylamin, *N*-Methylmorpholin, Pyridin und Tetramethylendiamin; Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendio-xid und Dimethylsulfoxid, Tetramethylensulfoxid, Dipropylsulfoxid, Benzylmethylsulfoxid, Diisobutylsulfoxid, Dibutylsulfoxid, Düsoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensul-fon; aliphatische, cycloaliphatische oder aromatische Kohlenwasserstoffe wie Pentan, Hexan, Hep-tan, Oktan, Nonan und technische Kohlenwasserstoffe; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohe-xan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N-*Methyl-formamid, *N*,*N-*Dimethyl-formamid, *N,N-*Dipropyl-formamid, *N,N-*Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formyl-piperidin, *N*,*N*'-1,4-Diformyl-piperazin; Ketone wie Aceton, Acetophenon, Methylethylketon, Methylbutylketon.

Verdünnungsmittel werden vorteilhaft in einer solchen Menge eingesetzt, dass das Reaktionsgemisch während des ganzen Verfahrens gut rührbar bleibt.

Bevorzugte Verdünnungsmittel zur Durchführung des erfindungsgemäßen Verfahrens 2 sind Ether wie Methyl-*tert*-butylether, *n*-Butylether, Anisol, Phenetol, Cyclohexylmethylether, Diisopropylether, Diisobutylether, Diisoamylether, Ethylenglycoldimethylether, Tetrahydrofuran, Dioxan, Dichlordiethylether und Polyether des Ethylenoxids und/oder Propylenoxids, Amide wie Hexamethylenphosphorsäuretriamid, Formamid, *N-*Methyl-formamid, *N,N-*Dimethyl-formamid, *N,N-*Dipropyl-formamid, *N,N*-Dibutyl-formamid, Benzol, Toluol, Chlorbenzol, Brombenzol, Nitrobenzol, Xylol; Ketone wie Aceton, Acetophenon, Methylethylketon oder Methylbutylketon.

Selbstverständlich kann man in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind jedoch Ether wie Methyl-*tert*-butylether oder cyclische Ether wie Tetrahydrofuran und Dioxan, Amide wie *N,N-*Dimethyl-formamid, aromatische Kohlenwasserstoffe wie Benzol oder Toluol; Ketone wie Aceton, Methylethylketon oder Methylbutylketon.

Als basische Reaktionshilfsmittel zur Durchführung des erfindungsgemäßen Verfahrens 2 können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Hydride, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo-(5.4.0)undecen (DBU), Cyclohexyltetrabutyl-guanidin (CyTBG), Cyclohexyltetramethylguanidin (CyTMG), *N,N,N,N-*Tetramethyl-1,8-naphthalindiamin, Pentamethylpiperidin, tertiäre Amine wie Triethylamin, Trimethylamin, Tribenzylamin, Trüsopropylamin, Tributylamin, Tricyclohexylamin, Triamylamin, Trihexylamin, *N,N*-Dimethylanilin, *N,N-*Dimethyl-toluidin, *N,N-*Dimethyl-*p-*aminopyridin, *N*-Methyl-pyrrolidin, *N*-Methyl-piperidin, *N-*Methyl-imidazol, *N*-Methyl-pyrazol, *N*-Methyl-morpholin, *N*-Methyl-hexamethylendiamin, Pyridin, 4-Pyrrolidinopyridin, 4-Dimethyl-amino-pyridin, Chinolin, α-Picolin, β-Picolin, Isochinolin, Pyrimidin, Acridin, *N,N,N',N'-Tetra-*methylendiamin, *N,N,N',N'*-Tetraethylendiamin, Chinoxalin, *N*-Propyl-diisopropylamin, N-Ethyldiisopropylamin, *N,N'*-Dimethyl-cyclohexylamin, 2,6-Lutidin, 2,4-Lutidin oder Triethyldiamin.

Vorzugsweise finden Hydride des Lithiums oder Natriums Verwendung.

Die Reaktionsdauer beträgt im Allgemeinen 10 Minuten bis 48 Stunden. Die Umsetzung erfolgt bei Temperaturen zwischen -10°C und +200°C, bevorzugt zwischen +10°C und 180°C, besonders bevorzugt zwischen 60°C und 140°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Verfahrens 2 setzt man pro Mol Verbindung der Formel (II) im Allgemeinen 0,5 bis 4,0 Mol, vorzugsweise 0,7 bis 3,0 Mol, besonders bevorzugt 1,0 bis 2,0 Mol an Alkylierungsmittel der Formel (IV) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Verfahren 3 zur Herstellung der neuen Verbindungen der Formel (I) in einem ersten Reaktionsschritt als Verbindungen der Formel (II) beispielsweise das 4-Hydroxyfuran-2(5*H*)-thion ein und als Verbindung der Formel (V) das 2-Fluorethylamin ein, und in einem zweiten Reaktionsschritt als entstandene Verbindung der Formel (VI) das 4-[(2-Fluor-ethyl)amino]furan-2(5H)-thion, welches mit Verbindungen der Formel (VII), beispielsweise 2-Chlor-5-(chlormethyl)pyridin *N*-alkyliert wird, so lässt sich das Herstellungsverfahren 3 durch folgendes Reaktionsschema VI wiedergeben:

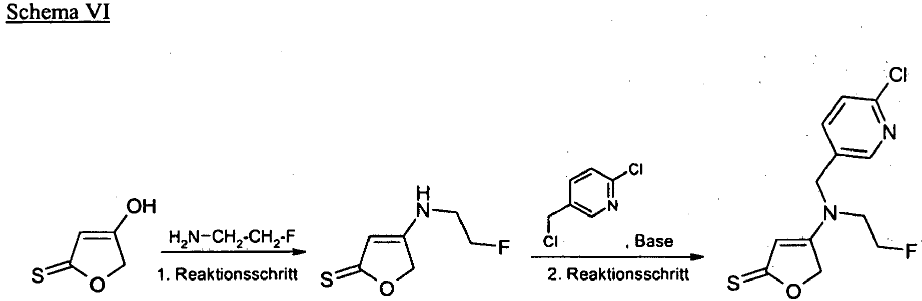

Die zur Durchführung des erfindungsgemäßen Verfahrens 3 als Ausgangsstoffe benötigten Verbindungen sind durch die Formel (II) allgemein definiert und bereits im weiter oben genannten Verfahren 1 näher beschrieben.

Die weiterhin für die Durchführung des erfindungsgemäßen Verfahrens 3 als Ausgangsstoffe zu verwendenden Verbindungen sind allgemein durch die Formel (V) definiert.

In der Formel (V) hat R¹ die Bedeutung, die bereits im Zusammenhang mit der Beschreibung der erfindungsgemäßen Stoffe der Formel (I) genannt wurde.

Die Aminoverbindungen der Formel (V) können in vielen Fällen z. T. kommerziell (vgl. z. B. 2-Fluorethylamin oder 2,2-Difluorethylamin) oder in an sich bekannter Weise nach der "Leuckart-Wallach-Reaktion (z B. 2-Fluor-ethylamin: US-Pat. 4030994 (1977); Verbindungen der Formel (V) in welcher R¹ für Alkyl steht, primäre Amine: vgl. z. B. Houben-Weyl, Methoden der Organischen Chemie, Bd. XI/1, 4. Aufl. 1957, Georg Thieme Verlag Stuttgart, S. 648; M. L. Moore in "The Leuckart Reaction" in: Organic Reactions, Bd. 5, 2. Aufl. 1952, New York, John Wiley & Sons, Inc. London) erhalten werden (vgl. z. B. auch 3-Fluor-*n*-propylamin: US 6252087 B1; 3,3-Difluor-prop-2-enylamin Hydrochlorid: WO 2001/007414 A1; 3,3-Dichlor-prop-2-enylamin: DE 2747814); 2-Chlor-2-fluor-cyclopropylamin, 2,2-Dichlor-cyclopropylamin: K. R. Gassen, B. Baasner, J. Fluorine Chem. 49, 127-139, 1990).

Alternativ können bestimmte Aminoverbindungen der Formel (Va), in welcher R¹ für CH₂-R' (R' = halogenhaltiger Rest; Halogen = Fluor oder Chlor) steht, auch durch Reduktion von halogenierten Carbonsäureamiden (VIII) in Gegenwart geeigneter Reduktionsmittel erhalten werden (Reakti-onsschema VII).

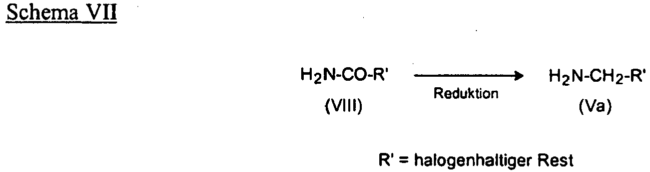

Als bevorzugtes Reduktionsmittel eignet sich beispielsweise der bekannte Boran-Dimethylsulfid Komplex (vgl. auch die Herstellung von 2-Chlor-2-fluorethan-1-amin aus kommerziell erhältlichem 2-Chlor-2-fluoracetamid).

Die weiterhin für die Durchführung des erfindungsgemäßen Verfahrens 3 als Ausgangsstoffe zu verwendenden Verbindungen sind allgemein durch die Formel (VII) definiert.

In der Formel (VII) haben E und A die Bedeutung, die bereits im Zusammenhang mit der Beschreibung der erfindungsgemäßen Stoffe der Formel (I) genannt wurde.

Die Verbindungen der allgemeinen Formel (VII) sind wie bereits weiter oben erwähnt z. T. kommerziell erhältlich, teilweise bekannt oder können nach bekannten Methoden erhalten werden.

Im Allgemeinen ist es vorteilhaft, den ersten Reaktionsschritt des erfindungsgemäßen Herstellungsverfahrens 3 in Gegenwart von Verdünnungsmitteln und gegebenenfalls in Gegenwart eines sauren Hilfsmittels umsetzt.

Als saure Hilfsmittel können beispielsweise die weiter oben, unter Verfahren 1 genannten sauren Hilfsmittel verwendet werden.

Verdünnungsmittel werden vorteilhaft in einer solchen Menge eingesetzt, dass das Reaktionsge-misch während des ganzen Verfahrens gut rührbar bleibt. Als Verdünnungsmittel zur Durchführung des erfindungsgemäßen Verfahrens 3 kommen alle inerten organischen Lösungsmittel in Frage.

Bevorzugte Verdünnungsmittel zur Durchführung des ersten Reaktionsschritts des erfindungsge-mäßen Verfahrens 3 sind aromatische Kohlenwasserstoffe wie Benzol, Toluol, Chlorbenzol, Brombenzol, Nitrobenzol oder Xylol, insbesondere aber Benzol und Toluol.

Besonders bevorzugt erfolgt die Herstellung von Verbindungen der Formel (I) nach dem Herstellungsverfahren 3, indem man zur Durchführung des ersten Reaktionsschritts Verbindungen der Formel (II) in Gegenwart von Verbindungen der Formel (V) in einem sauren Hilfsmittels, das gleichzeitig ein Verdünnungsmittel ist, umsetzt.

Als geeignetes saures Hilfsmittels, das gleichzeitig Verdünnungsmittel ist, kann beispielsweise Essigsäure verwendet werden.

Die Reaktionsdauer beträgt im Allgemeinen 10 Minuten bis 20 Tage.

Im zweiten Reaktionsschritt werden die Verbindungen der Formel (VI) mit Verbindungen der Formel (VII) N-alkyliert.

Im Allgemeinen ist es vorteilhaft, den zweiten Reaktionsschritt des erfindungsgemäßen Herstellungsverfahrens 3 in Gegenwart von Verdünnungsmitteln und in Gegenwart von basischen Reaktionshilfsmitteln wie beispielsweise Natriumhydrid durchzuführen.

Als Verdünnungsmittel für diesen Reaktionsschritt kommen beispielsweise Ether wie Tetrahydrofuran oder Dioxan in Frage.

Verdünnungsmittel werden vorteilhaft in einer solchen Menge eingesetzt, dass das Reaktionsgemisch während des ganzen Verfahrens gut rührbar bleibt.

Die Reaktionsdauer beträgt im Allgemeinen 10 Minuten bis 48 Stunden.

Die Umsetzung erfolgt bei Temperaturen zwischen -10°C und +200°C, bevorzugt zwischen +10°C und 180°C, besonders bevorzugt zwischen 60°C und 140°C. Man arbeitet bevorzugt unter Reaktionsbedingungen, die das Abscheiden oder Entfernen von Wasser ermöglichen, beispielsweise unter Zuhilfenahme eines Wasserabscheiders.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Alternativ und gegebenenfalls können zur Herstellung der neuen Verbindungen der Formel (I) worin A, B und R¹ bis R³ die weiter oben genannten Bedeutungen haben, auch die Verbindungen der Formel (IX) als Vorstufen in Gegenwart eines geeigneten Schwefelungsreagenz gemäß Schema VIII eingesetzt werden:

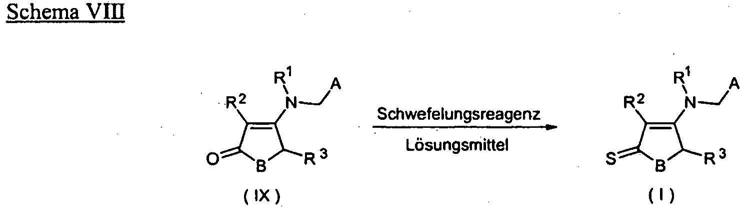

Die Verbindungen der allgemeinen Formel (IX) sind bekannt und können gemäß EP 0 539 588 A1, WO 2007/115643, WO 2007/115644 oder WO 2007/115646 erhalten werden.

Eine Vielzahl unterschiedlicher Schwefelungsreagenzien sind in der Literatur beschrieben, wie beispielsweise Schwefelwasserstoff (H₂S), Schwefelwasserstoff/Chlorwasserstoff (H₂S/HCl), wasserstoffpersulfid/Chlorwasserstoff (H₂S₂/HCl), Di(diethylaluminium)-sulfid [(Et₂Al)₂S], polymeres Ethylaluminiumsulfid [(EtAlS)ₙ], Siliziumdisulfid (SiS₂), Dibortrisulfid (B₂S₃), Phosphorpentachlorid/Dialuminiumtrisulfid/Natriumsulfat (PClₛ/Al₂S₃/Na₂SO₄), Natriumsulfid/Schwefelsäure (Na₂S/H2SO₄) Diphosphorpentasulfid (P₂S₅), Diphosphorpentasulfid/Pyridin (P₂S₅/Py), Diethylthi-ocarbamoylchlorid, Diphosphorpentasulfid/Triethylamin (P₂S₅/NEt₃), Diphosphorpentasulfid/n-Butyllithium (P₂S₅/nBuLi), Diphosphorpentasulfid/Natriumhydrogencarbonat (P₂S₅/NaHCO₃ "Scheeren's Reagenz, bildung von Na²⁺ [P₄S₁₀O]²⁻), Diphosphorpentasulfid/Methanol (P₂S₅/MeOH), SCN-COOEt, PSClₓ (NMe₂)₃₋ₓ (X = 0-3), Bis(1,5-cyclooktandiylboryl)sulfid [(9-BBN)₂S] als Schwefelungsreagens oder als Phosphorpentasulfid-Ersatz, 2,4-Bis(methylthio)-1,3,2,4-dithiadiphosphetan-2,4-disulfid "Davy-Reagens Methyl" (DR-Me), 2,4-Bis(ethylthio)-1,3,2,4-dithiadiphosphetan-2,4-disulfid "Davy-Reagens Ethyl" (DR-Et), 2,4-Bis(*p*-tolylthio)-1,3,2,4-dithiadiphosphetan-2,4-disulfid "Davy-Reagens *p*-Tolyl oder Heimgartner Reagens" (DR-T), 2,4-Bis(4-phenoxyphenyl)-2,4-dithioxo-1,3,2,4-dithiadiphosphetan "Belleau's Reagens (BR)", 2,4-Bis(4-methoxyphenyl)-2,4-dithioxo-1,3,2,4-dithiadiphosphetan "Lawesson's Reagens (LR)" (vgl. Davy-Reagens: H. Heimgartner et a/., Helv. Chim. Acta 70, 1987, S. 1001; Belleau's Reagens: Tetrahedron 40, 1984, S. 2047; Tetrahedron 40, 1984, S. 2663; Tetrahedron Letters 24, 1983, S. 3815; I. Thomson et al., Org. Synth. 62, 1984, S. 158 sowie die dort zitierte Literatur; D. Brillon Synth. Commun. 20, 1990, S. 3085 und die dort zitierte Literatur; selektive Thionierung von Oligopeptiden: K. Clausen et al., J. Chem. Soc., Perkin Trans. I 1984, S. 785; O. E. Jensen et al., Tetrahedron 41, 1985, S. 5595; "Lawesson's Reagens (LR)": R. A. Cherkasov et al., Tetrahedron 41, 1985, S. 2567; K. Clausen et. al., Tetrahedron 37, 1981, S. 3635; M. P. Cava et al., Tetrahedron 41, 1985, s. 5061; Diborylsulfid: Liebigs Ann. Chem. 1992, S. 1081 und die dort zitierte Literatur; Metzner et al., in Sulfur Reagents in Organic Synthesis, B. Harcourt (ed.), Academic Press, London, 1994, S. 44-45).

Alternativ sind auch Reaktionsfolgen, wie beispielsweise eine O-Alkylierung mit R₃O⁺BF⁴⁻ (R = Methyl, Ethyl) (H. Meerwein et al., Justus Liebigs Ann. Chem. 641, 1961, S. 1) und anschließende Umsetzung der Intermediate mit wasserfreiem NaSH (R. E. Eibeck, Inorg. Synth. 7, 1963, S. 128), die *in-situ* Bildung von Chloriminiumsalzen und nachfolgende Reaktion mit Tetrathiomolybdaten, insbesondere Benzyltriethylammoniumtetramolybdat [(Ph-CH₂-NEt₃)₂MoS₄] *(*Tetrahedron Lett. 36, 1995, S. 8311) oder Hexamethyldisilathian (TMS₂S) (TMS = Trimethylsilyl; P. L. Fuchs et al., J. Org. Chem. 59, 1994, S. 348) möglich.

Als Sulfidierungsmittel werden bevorzugt Phosphorreagenzien wie beispielsweise Diphosphorpentasulfid (P₂S₅), Diphosphorpentasulfid/Pyridin (P₂S₅/Py), Diphosphorpentasulfid/Triethylamin (P₂S₅/NEt₃), Diphosphorpentasulfid/Natriumhydrogencarbonat (P₂S₅/NaHCO₃ "Scheeren's Reagenz) oder besonders bevorzugt das 2,4-Bis(4-methoxyphenyl)-2,4-dithioxo-1,3,2,4-dithiadiphosphetan "Lawesson's Reagens (LR)", 2,4-Bis(4-phenoxyphenyl)-2,4-dithioxo-1,3,2,4-dithiadiphosphetan "Belleau's Reagens (BR)" bzw. 2,4-Bis(4-phenylthiophenyl)-2,4-dithioxo-1,3,2,4-dithiadiphosphetan, eingesetzt.

Zur Darstellung der Verbindungen der Formel (I) in der R² für Halogen steht, können alternativ auch Verbindungen der Formel (I) in der R² für Wasserstoff steht, mit Halogenierungsmitteln in Gegenwart von basischen Hilfsmitteln gemäß dem Reaktionsschema (IX) umgesetzt werden.

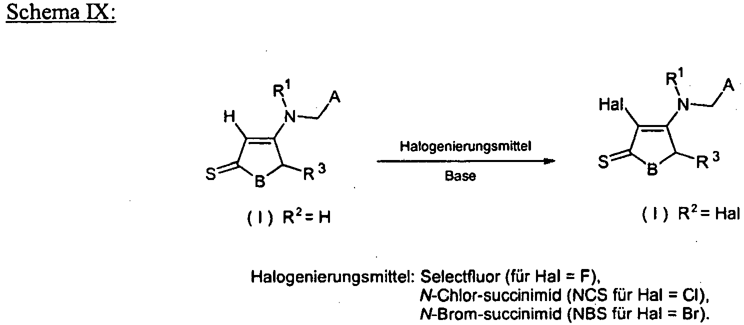

In den als Ausgangsstoffe benötigten Verbindungen der Formel (I) haben A, B, R¹ und R³ die weiter oben genannte Bedeutung, der Substituent R² steht für Wasserstoff.

Diese Verbindungen der Formel (I) können nach den weiter oben genannten Herstellverfahren 1 bis 3 erhalten werden.

Im Allgemeinen ist es vorteilhaft, die Halogenierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der Halogenierung kommen alle unter den Reaktionsbedingungen inerten organischen Lösungsmittel in Frage.

Als Halogenierungsmittel zur Durchführung des erfindungsgemäßen Verfahrens können alle geeigneten Halogenierungsmittel eingesetzt werden, beispielsweise *N*-Halogen-Verbindungen.

Beispielhaft seien genannt N-Halogenamine wie 1-Chlormethyl-4-fluordiazoniabicyclo[2.2.2]-octan-bis-(tetrafluoroborat) (Selectfluor^{®}), *N,N*-Dihalogenamine, *N*-Halogen-carbonsäureamide, *N-*Halogen-carbamidsäureester, *N*-Halogen-hamstoff, *N*-Halogen-sulfonylamide, *N*-Halogen-disulfonylamide, *N*-Halogen-sulfonylimide wie *N*-Fluor-bis[(trifluorrnethyl)sulfonyl]imid und *N-*Halogen-carbonsäurediamide wie *N*-Chlor-phthalimid, *N*-Brom-phthalimid, *N*-Iod-phthalimid, *N-*Chlor-succinimid (NCS), N*-*Brom-succinimid (NBS), *N*-Brom-saccharin oder *N-*Iod-succinimid.

Bevorzugte Halogenierungsmittel zur Durchführung der Halogenierung sind die *N*-Halogen-carbonsäurediamide oder 1-Chlormethyl-4-fluordiazoniabicyclo[2.2.2]octan-bis-(tetrafluoroborat) (Selectfluor^{®}).

Bevorzugte Verdünnungsmittel zur Durchführung der Halogenierung sind Nitrile wie Acetonitril, Propionitril, Butyronitril, Isobutyronitril, Benzonitril, m-Chlorbenzonitril.

Man kann in das erfindungsgemäße Verfahren auch Gemische der genannten Lösungs- und Verdünnungsmittel einsetzen.

Besonders bevorzugte Verdünnungsmittel zur Durchführung des erfindungsgemäßen Verfahrens sind Nitrile wie Acetonitril, Propionitril oder Butyronitril.

Die Reaktionsdauer bei diesem Verfahren beträgt im Allgemeinen 10 Minuten bis 48 Stunden.

Die Umsetzung erfolgt bei Temperaturen zwischen -10°C und +100°C, bevorzugt zwischen 0°C und 60°C, besonders bevorzugt zwischen 10°C und Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryöbia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hytotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanüs, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.,

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomrna livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Steplianitis nashi, Tibraca spp., Triatoma spp.

Aus der Ordnung der Homoptera z.B. Acyrthosipon spp., Aeneolamia spp., Agonoscena spp., A-leurodes spp., Aleurolobus barodensis, Aleurothrixus spp., Amrasca spp., Anuraphis cardui, Aoni-diella spp., Aphanostigma piri, Aphis spp., Arboridia apicalis, Aspidiella spp., Aspidiotus spp.; Atanus spp., Aulacorthum solani, Bemisia spp., Brachycaudus helichrysii, Brachycolus spp., Brevicoryne brassicae, Calligypona marginata, Cameocephala fulgida, Ceratovacuna lanigera, Cercopidae, Ceroplastes spp., Chaetosiphon fragaefolii, Chionaspis tegalensis, Chlorita onukii, Chro-maphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabe-misia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopälosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotisalbicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenem, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch einoder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CrylA(c), CryIIA, CryIIIA, CryI-IIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "bit Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Mäissorten, Baumwollsorten und Sojasorten genannt, die unter den Handelsbezeichnungen Roundup Ready®, (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggem, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel 1

### 4-{[(6-Chlorpyridin-3-yl)methyl](2,2-difluoroethyl)amino}furan-2(5H)-thion

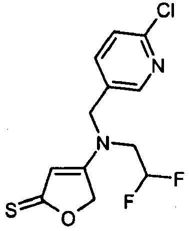

20 mg (0,169 mMol) 4-Hydroxyfuran-2(5*H*)-thion (bekannt aus: R. Labruère et al., Synthesis 4163-4166, 2006) in 0.5 mL Essigsäure wurden mit 105 mg (0,506 mMol) *N*-[(6-chlorpyridin-3-yl)methyl]-2,2-difluorethanamin (herstellbar gemäß: WO 2007/115644 A1, WO 2008/009360 A2) versetzt und 14 Tage bei Raumtemperatur gerührt. Einengen im Vakuum und Reinigung des Rückstandes mittels präparativer HPLC (RP18, CH₃CN-H₂O) lieferten 15 mg (Ausbeute: 28.8 % der Theorie) 4-{[(6-Chlorpyridin-3-yl)methyl](2,2-difluoroethyl)amino}furan-2(5H)-thion.
¹H-NMR (CDCl₃): δ [ppm] = 3.54 (td, 2 H), 4.53 (s, 2 H), 5.18 (s, 2 H), 5.75 (s, 1 H), 5.97 (t, 1 H), 7.40 (d, 1 H), 7.53 (dd, 1 H), 8.29 (d, 1 H).
¹³C-NMR (CDCl₃): δ [ppm] = 52.6, 53.4, 75.0, 104.2 (br), 112.9 (br), 125.0, 128.4, 137.4, 148.5, 152.2, 169.5 (br), 212.3.

### Bioloeische Beispiele

### Beispiel Nr. 1

### Phaedon-Test (PHAECO Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigte z. B. die folgende Verbindung der Herstellungsbeispiele eine Wirkung von ≥ 80 % bei einer Aufwandmenge von 500 g/ha: Bsp. Nr. 1

### Beispiel Nr. 2

### Myzus-Test (MYZUPE Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblättlaüs *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigte z. B. die folgende Verbindung der Herstellungsbeispiele eine Wirkung von ≥ 80 % bei einer Aufwandmenge von 500 g/ha: Bsp. Nr. 1

## Patentansprüche

1. Verbindungen der Formel (I) 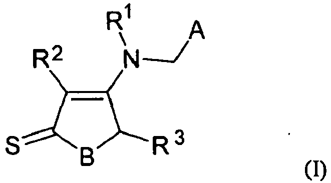 in welcher
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy steht, oder A für einen Rest der folgenden Formel steht 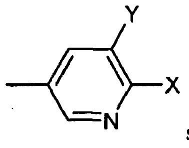 steht, in welchem
X für Halogen, Alkyl oder Halogenalkyl steht, und
Y für Halogen, Alkyl, Halogenalkyl, Halogenalkoxy, Azido oder Cyan steht;
B für Sauerstoff steht;
R¹ für Halogenalkyl oder Halogencycloalkyl steht,
R² für Wasserstoff steht und
R³ für Wasserstoff steht.

2. Mittel, **gekennzeichnet durch** einen Gehalt von mindestens einer Verbindung der Formel (I) gemäß Anspruch 1 und üblichen Streckmitteln und/oder oberflächenaktiven Substanzen.

3. Verfahren zum Bekämpfen von Schädlingen **dadurch gekennzeichnet, dass** man eine Verbindung der Formel (I) gemäß Anspruch 1 oder ein Mittel gemäß Anspruch 2 auf die Schädlinge und/oder ihren Lebensraum einwirken lässt.

4. Verwendung von Verbindungen der Formel (I) gemäß Anspruch 1 oder von Mitteln gemäß Anspruch 2 zur Bekämpfung von Schädlingen.

## Claims

1. Compounds of the formula (I) 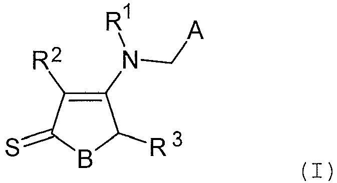 in which
A represents pyrid-2-yl or pyrid-4-yl or represents pyrid-3-yl which is optionally substituted in the 6-position by fluorine, chlorine, bromine, methyl, trifluoromethyl or trifluoromethoxy, or A represents a radical of the formula below 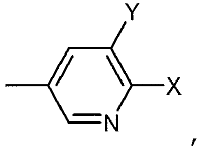 in which
X represents halogen, alkyl or haloalkyl, and
Y represents halogen, alkyl, haloalkyl, haloalkoxy, azido or cyano;
B represents oxygen;
R¹ represents haloalkyl or halocycloalkyl,
R² represents hydrogen and
R³ represents hydrogen.

2. Composition, **characterized in that** it comprises at least one compound of the formula (I) according to Claim 1 and customary extenders and/or surfactants.

3. Method for controlling pests, **characterized in that** a compound of the formula (I) according to Claim 1 or a composition according to Claim 2 is allowed to act on the pests and/or their habitat.

4. Use of compounds of the formula (I) according to Claim 1 or of compositions according to Claim 2 for controlling pests.

## Revendications

1. Composés de formule (I) 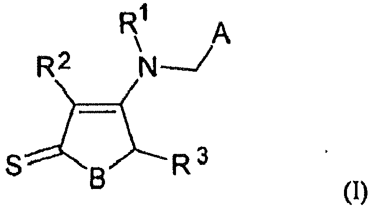 dans laquelle
A représente pyrid-2-yle ou pyrid-4-yle ou pyrid-3-yle, qui est éventuellement substitué en position 6 par fluor, chlore, brome, méthyle, trifluorométhyle ou trifluorométhoxy, ou A représente un radical de la formule suivante 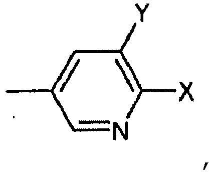 dans laquelle
X représente halogène, alkyle ou halogénoalkyle, et
Y représente halogène, alkyle, halogénoalkyle, halogénoalcoxy, azido ou cyano ;
B représente oxygène ;
R¹ représente halogénoalkyle ou halogénocycloalkyle,
R² représente hydrogène et
R³ représente hydrogène.

2. Agent, **caractérisé par** une teneur en au moins un composé de formule (I) selon la revendication 1 et des extendeurs et/ou substances tensioactives usuels.

3. Procédé de lutte contre les animaux nuisibles, **caractérisé en ce qu'**un composé de formule (I) selon la revendication 1 ou un agent selon la revendication 2 est laissé agir sur les animaux nuisibles et/ou leur habitat.

4. Utilisation de composés de formule (I) selon la revendication 1 ou d'agents selon la revendication 2 pour lutter contre les animaux nuisibles.
